(19) Europäisches Patentamt

European Patent Office

Office européen des brevets

(11) Publication number: **0 294 200 B1**

# EUROPEAN PATENT SPECIFICATION

(45) Date of publication of patent specification: **15.04.92**　(51) Int. Cl.⁵: **A61M 16/04**

(21) Application number: **88305042.9**

(22) Date of filing: **02.06.88**

(54) **Artificial airway device.**

(30) Priority: **05.06.87 GB 8713173**

(43) Date of publication of application:
**07.12.88 Bulletin 88/49**

(45) Publication of the grant of the patent:
**15.04.92 Bulletin 92/16**

(84) Designated Contracting States:
**AT BE CH DE ES FR GR IT LI LU NL SE**

(56) References cited:
FR-A- 2 517 974　　GB-A- 1 186 964
US-A- 2 099 127　　US-A- 4 227 534
US-A- 4 324 235　　US-A- 4 502 482

(73) Proprietor: **Brain, Archibald Ian Jeremy, Dr.**
**10 Preston Drive**
**Wanstead London E11(GB)**

(72) Inventor: **Brain, Archibald Ian Jeremy, Dr.**
**10 Preston Drive**
**Wanstead London E11(GB)**

(74) Representative: **Sawers, Lawrence Peter et al**
**PAGE, WHITE & FARRER 54 Doughty Street**
**London WC1N 2LS(GB)**

## Description

This invention relates to an artificial airway device to facilitate lung ventilation in an unconscious patient, and more specifically to such a device designed for placing in the oropharynx of the patient in order to prevent airway obstruction, to permit either spontaneous or controlled ventilation and to prevent the inhalation into the lungs of extraneous matter such as blood or vomit.

To maintain the airway of an unconscious patient, and to achieve the three objectives mentioned above, it is normal practice in general anaesthesia to use an endotracheal tube, which is a flexible tube of rubber or plastics, usually with an inflatable cuff around the distal end. Alternatively, an oro- or naso-pharyngeal airway may be used, which is a flexible tube extending from the mouth or nose into the pharynx but not into the larynx, and which is used in conjunction with a face mask, unlike the endotracheal tube. While preventing obstruction of the airway by the tongue, the oro- or naso-pharyngeal airway cannot conveniently be used for controlled ventilation and does not prevent inhalation of extraneous matter.

The endotracheal tube is introduced through the larynx into the trachea or windpipe, whereupon the cuff is inflated through a small auxiliary tube to seal against the wall of the trachea. Introduction of the endotracheal tube is a skilled operation normally requiring use of a laryngoscope to guide the tube through the larynx, past the vocal cords and into the trachea. There is a risk that the tube or the laryngoscope may cause damage to soft tissues or to the sensitive structures of the larynx. It is not always possible to see the larynx, making intubation difficult or impossible in some patients. There can be a risk of accidental intubation of the oesophagus or of the right or left main bronchus. Placing of the tube in the trachea effectively narrows the interior passage or lumen of the trachea and provides a potential source of damage through infection or pressure while preventing normal upward flow of mucus from the trachea and rendering effective coughing impossible.

US 2099127 describes a pharyngeal bulb gasway which has two metal loops projecting from the end of the gasway to prevent the epiglottis or other tissue from closing over the end of the gasway. When in use the gasway is positioned within the pharynx so as to extend anteriorly and the metal loops press into the base of the tongue and urge the epiglottis away from the base of the tongue. In this manner the airway is kept clear.

In my British Patent Specification No. 2111394B, I have described and claimed an artificial airway device comprising a curved or flexible tube and a mask portion carried at one end of the tube, the mask portion having a flexible annular peripheral formation which may be inflatable and which surrounds a hollow interior space or lumen of the mask portion, said annular peripheral formation of the mask portion being pre-formed with a roughly elliptical shape such as to be capable of conforming to, and of fitting readily within, the actual and potential space behind the larynx so as to form a seal around the circumference of the laryngeal inlet without the device penetrating into the interior of the larynx, the tube opening into the lumen of the mask portion to provide the airway with the axis of the tube substantially aligned with the length of the roughly elliptical annular peripheral formation of the mask portion. The device thus constitutes a laryngeal mask.

This device has proved successful in use. Insertion of the device has been found to be easy and convenient in the majority of patients. A laryngoscope is not usually required. The mask does not enter the larynx or trachea so the risk of damage to these structures is avoided and the tracheal lumen is not narrowed as it is by insertion of an endotracheal tube. The risk of accidental entry into the oesophagus or one of the main bronchi is also avoided. Once in place the laryngeal mask generally permits the lungs to be ventilated by positive pressure. Alternatively the patient may be permitted to breathe spontaneously.

On occasion, however, it has been found that the epiglottis can obstruct the airway by falling inwards into the lumen of the mask and blocking the opening of the tube therein. This can happen, for example, with small displacements of the mask which may occur during surgery or manipulation of the patient on the operating table. An object of the present invention is to provide an improved airway device which will not be liable to such obstruction.

According to the present invention, an artificial airway device to facilitate lung ventilation in an unconscious patient comprises a curved or flexible airway tube and a mask carried at one end of the airway tube, the mask having a flexible annular peripheral formation of roughly elliptical shape capable of conforming to, and of readily fitting within, the actual and potential space behind the larynx so as to form a seal around the circumference of the laryngeal inlet without the device penetrating into the interior of the larynx, the annular peripheral formation surrounding a hollow interior space or lumen of the mask and the airway tube opening into the lumen of the mask with the axis of the airway tube substantially in the same plane as the major axis of the peripheral formation, characterised in that the airway tube opens into the lumen through an aperture across which extend one or more flexible cross-bars which are arranged to retain the epiglottis anteriorly so as to prevent it from obstructing the apperture while permitting passage of a second, smaller tube, when required.

Preferably, there are two, flexible cross-bars extending across the aperture, preferably substantially parallel with the major axis of the peripheral formation, and leaving the middle of the aperture clear for passage of a second, smaller tube as referred to above. It is preferred that the cross-bars extend substantially parallel with the major axis of the peripheral formation, since displacements of the mask mainly take place in this direction. In a preferred example, the spaces between the cross-bars and between the edges of the aperture and the adjacent cross-bars are substantially 3 to 4 mm in width in a mask designed for use with a normal adult patient. These dimensions ensure that the spaces will not normally be blocked by secretions but are not so large as to require the cross-bars to be made too thin.

The airway tube preferably opens into the lumen at an angle of close to 30° to the plane of the annular peripheral formation. This angle is important to ensure that any smaller tube passed through the airway tube and the mask will emerge at the correct angle for intubation of the larynx Such a tube may be, for example, an endotracheal or endobronchial tube or a suction catheter, or an inspection tube such as a fibre-optic broncho- or laryngoscope.

Introduction of the airway device may be facilitated in some difficult patients by use of a substantially rigid introducing tool which is subsequently withdrawn. Withdrawal of the introducing tool serves the additional purpose of automatically elevating the epiglottis, which may in such cases otherwise block the airway by being deflected downwards when the mask is introduced. To facilitate introduction of the tool, a flexible web closing the rear of the lumen of the mask is preferably provided with a pocket having a transverse slit opening towards the airway tube aperture, for receiving the end of the introducing tool.

As in the case of the device of my prior British Patent No. 2111394B, the annular peripheral portion of the mask is preferably inflatable.

A specific embodiment of the invention will now be described in more detail by way of example and with reference to the accompanying drawings, in which:-

Figure 1 is a side view of an artificial airway device in the form of a laryngeal mask,
Figure 2 is a plan view of the device with the periphery of the mask portion inflated,
Figure 3 is a section on the line III-III of Figure 2, and
Figure 4 shows diagrammatically the device in position for use in a patient.

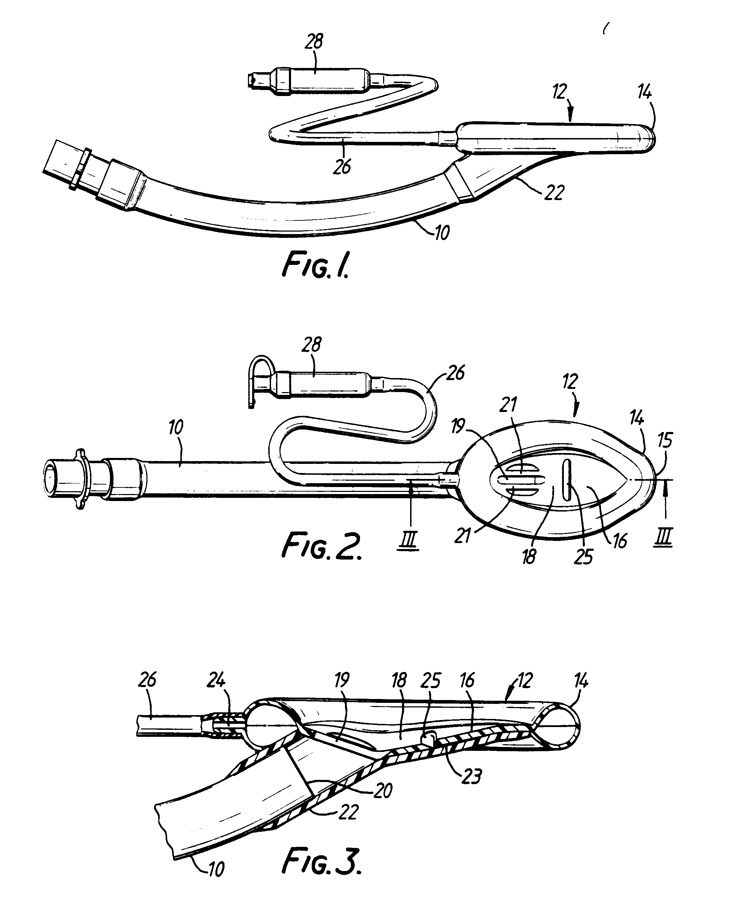

The laryngeal mask illustrated in the drawings comprises a flexible airway tube 10 of silicone rubber material, similar to that used for some endotracheal tubes, and a mask 12 of flexible silicone rubber sheet material, having an inflatable tubular ring 14 of the same silicone rubber material forming its periphery and a web 16 which closes off the rear of the interior or lumen 18 of the mask and is formed with an aperture 19. The distal end 20 of the airway tube 10 is secured, as by welding, in one end of a short piece 22 of thick-walled silicone rubber tubing whose other end is moulded to fit against the outer edge of the web 16 and around the aperture 19, so as to form a semi-rigid backpiece for the mask, which backpiece carries the airway tube 10 at an angle of substantially 30° to the plane of the ring 14. The airway tube 10 thus opens into the interior or lumen 18 of the mask 12 through the piece 22 and the aperture 19. The peripheral ring 14 is of roughly elliptical shape as seen in plan (Figure 2) though its distal end 15 may be slightly elongated to conform with the triangular shape of the base of the hypopharynx where it becomes continuous with the upper end of the oesophagus. The airway tube 10 lies in substantially the same plane as the major axis of the peripheral ring 14 and at substantially 30° to the plane of the ring 14. The ring 14 is formed with a port 24 into which is sealed one end of a flexible silicone rubber tube 26 of much smaller diameter. The other end of tube 26 is provided with an inflation indicator 28, and can be connected to a small pump (not shown) such as a disposable 20 ml medical syringe for inflation of the ring 14. Alternatively the tube 26 may be permanently connected through a valve to a collapsible bulb whose capacity is equal to the optimal inflated capacity of the ring 14.

The aperture 19 through which the airway tube 10 opens into the lumen 18 of the mask 12 is provided with two flexible cross-bars 21 extending across the aperture 19 substantially parallel with the major axis of the Peripheral ring 14, so as to leave the middle of the aperture clear for passage of an inspection or other tube. The bars 21 effectively prevent the epiglottis from falling into the aperture 19 and obstructing the airway.

For introduction of the device, a flat, longitudinally curved metal introducing tool (not shown) may be used in difficult cases, as explained above. To accommodate the leading edge of the tool, a pocket 23 is formed between the web 16 and the adjacent distal end of the piece 22, with a transverse slit 25 cut in the web 16 to provide an opening into the pocket 23 facing towards the airway tube aperture 19.

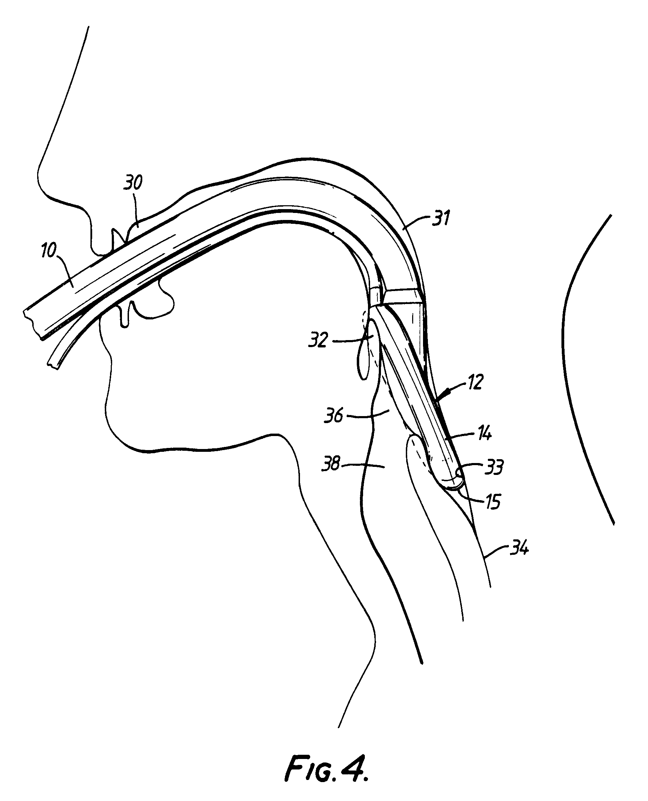

Different sizes of mask are needed for different sizes of patient. In use, the device is inserted as shown in Figure 4 through the patient's mouth 30 and down through the throat 31 past the epiglottis 32 until the mask 12 comes to rest with the distal end 15 of the ring 14 in the base 33 of the throat, lying against the upper end of the normally closed oesophagus 34, which the mask cannot easily enter provided that the correct size has been chosen. The ring 14 is then inflated as shown to increase the sealing pressure around the inlet 36 to the larynx 38. The patient's airway is thus secure and unobstructed and the laryngeal mask can be connected directly to conventional anaesthetic circuit hosing for either positive pressure or spontaneous breathing. As can be seen from Fig. 4, the airway tube 10 opens into the lumen of the mask 12 at the appropriate angle (substantially 30°) to enable an inspection or other tube (not shown) passed through the airway tube 10 and the mask 12 to emerge at the correct angle for intubation of the larynx 38.

In a modification (not shown) the back of the mask 12 may be formed with a longitudinal groove or recess to accommodate a further tube which may be introduced through the oesophagus to allow passage of gastric contents.

The embodiment described above may be used as a disposable instrument or as a re-usable one.

The tube and mask portion could also be made of other sterilisable materials, such as plastics. The materials may be more rigid than the inflatable silicone rubber materials described above. With some materials it may not be necessary that the peripheral ring should be inflatable. For example, the ring 14 may consist of a foam material within an air-tight covering, from which the air is evacuated to facilitate insertion of the mask. In any case the mask 12 will be shaped as described above to conform to and fit readily into the actual and potential space behind the larynx and to seal around the laryngeal inlet. The reference to actual and potential space will be understood to refer to the space normally available and that which can become available on flexure of the surrounding structures.

## Claims

1. An artificial airway device to facilitate lung ventilation in an unconscious patient, comprising a curved or flexible airway tube (10) and a mask (12) carried at one end of the airway tube (10), the mask (12) having a flexible annular peripheral formation (14) of roughly elliptical shape capable of conforming to, and of readily fitting within, the actual and potential space behind the larynx (38) so as to form a seal around the circumference of the laryngeal inlet (36) without the device penetrating into the interior of the larynx (38), the annular peripheral formation (14) surrounding a hollow interior space or lumen (18) of the mask (12) and the airway tube (10) opening into the lumen (18) of the mask (12) with the axis of the airway tube (10) substantially in the same plane as the major axis of the peripheral formation (14), characterised in that the airway tube (10) opens into the lumen (18) through an aperture (19) across which extend one or more flexible cross-bars (21) which are arranged to retain the epiglottis (32) anteriorly so as to prevent it from obstructing the apperture (19) while permitting passage of a second, smaller tube, when required.

2. An artificial airway device according to claim 1 characterised in that there are two of said flexible cross-bars (21).

3. An artificial airway device according to claim 1 or 2 characterised in that the cross-bar or cross-bars (21) is or are substantially parallel with the major axis of the peripheral formation (14) and leave the middle of the aperture (19) clear for passage of a second, smaller tube.

4. An artificial airway device according to any preceding claim characterised in that the spaces between the cross-bars (21) and between the edges of the aperture (19) and the adjacent cross-bars (21) are substantially 3 to 4 mm in width.

5. An artificial airway device according to any one of the preceding claims characterised in that the airway tube (10) opens into the lumen (18) at an angle of close to 30° to the plane of the annular peripheral formation (14).

6. An artificial airway device according to any one of the preceding claims characterised in that a flexible web (16) closing the rear of the lumen (18) of the mask (12) is provided with a pocket (23) having a transverse slit (25) opening towards the airway tube aperture (19), for receiving the end of a substantially rigid introducing tool.

7. An artificial airway device according to any one of the preceding claims characterised in that the annular peripheral formation (14) of the mask (12) is inflatable.

8. An artificial airway device according to claim 8, characterised in that the annular peripheral formation (14) is connected through a valve to a collapsible bulb whose capacity is equal to the optional inflated capacity of the peripheral formation (14).

## Revendications

1. Dispositif de respiration artificielle pour faciliter l'oxygénation des poumons d'un patient sans connaissance, comprenant un tuyau d'aspiration courbé ou souple (10) et un masque (12) supporté à une extrémité du tuyau d'aspiration (10), le masque (12) présentant une formation périphérique souple annulaire (14) d'une forme grossièrement elliptique pouvant se conformer et s'adapter facilement à l'intérieur de l'espace actuel et potentiel derrière le larynx (38) de façon à former une obstruction autour de la circonférence de l'entrée du larynx (36) sans que le dispositif pénètre à l'intérieur du larynx (38), la formation périphérique annulaire (14) entourant un espace ou passage intérieur creux (18) du masque (12) et le tuyau d'aspiration (10) s'ouvrant dans le passage (18) du masque (12) avec l'axe du tuyau d'aspiration (10) pratiquement dans le même plan que l'axe principal de la formation périphérique (14), caractérisé en cc que le tuyau d'aspiration (10) s'ouvre dans le passage (18) par l'intermédiaire d'une ouverture (19) à travers laquelle s'étend une ou plusieurs traverses souples (21) qui sont disposées pour retenir l'épiglotte (32) à l'avant de façon à l'empêcher d'obstruer l'ouverture (19) tout en permettant le passage d'un second tube d'un diamètre plus petit si nécessaire.

2. Dispositif de respiration artificielle selon la revendication 1, caractérisé en ce qu'il comporte deux desdites traverses souples (21).

3. Dispositif de respiration artificielle selon la revendication 1 ou 2, caractérisé en ce que la traverse ou les traverses (21) est ou sont pratiquement parallèles à l'axe principal de la formation périphérique (14) et laissent le milieu de l'ouverture (19) libre pour le passage d'un second tube d'un diamètre plus petit.

4. Dispositif de respiration artificielle selon l'une quelconque des revendications précédentes, caractérisé en ce que les espaces entre les traverses (21) et entre les bords de l'ouverture (19) et les traverses adjacentes (21) sont pratiquement d'une largeur de 3 à 4 mm.

5. Dispositif de respiration artificielle selon l'une quelconque des revendications précédentes, caractérisé en ce que le tuyau d'aspiration (10) s'ouvre dans le passage (18) à un angle proche de 30° par rapport au plan de la formation périphérique annulaire (14).

6. Dispositif de respiration artificielle selon l'une quelconque des revendications précédentes, caractérisé en ce qu'un tissu souple (16) fermant l'arrière du passage (18) du masque (12) est prévu avec une poche (23) comportant une fente transversale (25) s'ouvrant vers l'ouverture du tuyau d'aspiration (19) afin de recevoir l'extrémité d'un outil d'introduction pratiquement rigide.

7. Dispositif de respiration artificielle selon l'une quelconque des revendications précédentes, caractérisé en ce que la formation périphérique annulaire (14) du masque (12) est gonflable.

8. Dispositif de respiration artificielle selon la revendication 8, caractérisé en ce que la formation périphérique annulaire (14) est raccordée par l'intermédiaire d'une soupape à un ballon repliable dont la capacité est égale à la capacité gonflée possible de la formation périphérique (14).

## Patentansprüche

1. Künstliche Luftweg-Intubationsvorrichtung zur Erleichterung der Lungenventilation bei einem bewußtlosen Patienten, welche folgende Teile umfaßt: ein gebogenes oder flexibles Intubationsrohr (10) und eine Maske (12), welche an einem Ende des Intubationsrohrs (10) angebracht ist, wobei die Maske (12) ein flexibles, ringförmiges Umfangsgebilde (14) von ungefähr elliptischer Form aufweist, das sich an den tatsächlichen und möglichen Raum hinter dem Kehlkopf (38) anpassen und bequem in diesen Raum hineinpassen kann, wobei eine Abdichtung um den Umfang des Kehlkopfeinlasses (36) ausgebildet wird, ohne daß die Vorrichtung in das Innere des Kehlkopfs (38) eindringt, wobei das ringförmige Umfangsgebilde (14) einen hohlen Innenraum oder Lumen (18) der Maske (12) umgibt und das Intubationsrohr (10) in das Lumen (18) der Maske (12) so eintritt, daß die Achse des Intubationsrohrs (10) im wesentlichen in derselben Ebene wie die Hauptachse des Umfangsgebildes (14) liegt, dadurch gekennzeichnet, daß das Intubationsrohr (10) in das Lumen (18) durch eine Öffnung (19) eintritt, über die sich ein oder mehrere flexible Querstäbe (21) erstrecken, die so angeordnet sind, daß sie den Kehldeckel (Epiglottis) (32) vorher zurückhalten und so verhindern, daß er die Öffnung (19) blockiert, während sie das Vorbeiführen eines zweiten, kleineren Rohres erlauben, sofern dies erforderlich ist.

2. Künstliche Luftweg-Intubationsvorrichtung nach Anspruch 1, dadurch gekennzeichnet, daß es zwei flexible Querstäbe (21) gibt.

3. Künstliche Luftweg-Intubationsvorrichtung nach Anspruch 1 oder Anspruch 2, dadurch gekennzeichnet, daß der Querstab oder die Querstäbe (21) im wesentlichen parallel zur Hauptachse des Umfangsgebildes (14) angeordnet ist bzw. sind und die Mitte der Öffnung (19) für das Durchführen eines zweiten, kleineren Rohres frei läßt bzw. lassen.

4. Künstliche Luftweg-Intubationsvorrichtung nach irgendeinem der vorangehenden Ansprüche, dadurch gekennzeichnet, daß die Zwischenräume zwischen den Querstäben (21) und zwischen den Kanten der Öffnung (19) und den benachbarten Querstäben (21) eine Weite von im wesentlichen 3 bis 4 mm aufweisen.

5. Künstliche Luftweg-Intubationsvorrichtung nach irgendeinem der vorangehenden Ansprüche, dadurch gekennzeichnet, daß das Intubationsrohr (10) in das Lumen (18) in einem Winkel nahe bei 30 ° zur Ebene des ringförmigen Umfangsgebildes (14) eintritt.

6. Künstliche Luftweg-Intubationsvorrichtung nach irgendeinem der vorangehenden Annsprüche, dadurch gekennzeichnet, daß ein flexibles Gewebe (16), welches die Rückseite des Lumens (18) der Maske (12) verschließt, mit einer Tasche (23) versehen ist, welche einen Schrägschlitz (25) aufweist, welcher in Richtung auf die Öffnung (19) des Intubationsrohrs gerichtet ist, um das Ende eines im wesentlichen starren Werkzeugs zum Einführen aufzunehmen.

7. Künstliche Luftweg-Intubationsvorrichtung nach irgendeinem der vorangehenden Ansprüche, dadurch gekennzeichnet, daß das ringförmige Umfangsgebilde (14) der Maske (12) aufblasbar ist.

8. Künstliche Luftweg-Intubationsvorrichtung nach Anspruch 7, dadurch gekennzeichnet, daß das ringförmige Umfangsgebilde (14) durch ein Ventil mit einem zusammenfaltbaren Ball verbunden ist, dessen Kapazität gleich der möglichen Kapazität des Umfangsgebildes (14) im aufgeblasenen Zustand ist.

FIG. 1.

FIG. 2.

FIG. 3.

EP 0 294 200 B1

FIG.4.